# EUROPEAN PATENT APPLICATION

(11) **EP 3 951 274 A1**
(43) Date of publication of application: **09.02.2022**
(21) Application number: 21183991.5
(22) Date of filing: 06.07.2021
(51) Int. Cl.: F24F 7/003, F24F 7/06, F24F 8/108, F24F 8/167, F24F 8/22, F24F 8/30, F24F 8/26, A61L 9/20, A61L 9/22

(54) **AIR PURIFYING APPARATUS**

(30) Priority: 06.08.2020 IT 202000019462
(71) Applicant: Nanoproject S.r.l., 40138 Bologna (IT)
(72) Inventor: LANZA, Roberto, 13058 PONDERANO (BI) (IT)
(74) Representative: Modiano, Micaela Nadia

(57) **Abstract**

An air purifying apparatus (1), of the type comprising a conveyance channel (2) intercepted by at least one filter (3, 4) which comprises: at least one first intake filter (3) arranged at the opening (5) for access to the conveyance channel (2); at least one duct (6) provided with an internal coating (7) constituted by a mixture which comprises at least one photocatalytic material; at least one LED diode (8), designed to emit a beam of light that is at least partially comprised in ultraviolet radiation within the duct (6); at least one cold plasma purification device (9), which has an outlet (10) which leads, even indirectly, to the terminal outflow opening (11) of the channel (2).

## Description

The present invention relates to an air purifying apparatus.

Due to pollution and to the increasingly frequent spread of allergies affecting the respiratory system of people, the use of devices for air purification is increasingly frequent.

These devices can be simply constituted by sets of filters which trap part of the particles suspended in the air.

More recently, some units designed to physically separate particles and capable of performing a bactericidal action have been coupled to the filters.

In particular, purification devices using cold plasma (or non-thermal plasma, in which, in other words, the electrons are not in thermodynamic equilibrium with the other species since they are characterized by a much higher temperature than the heavier species, i.e., ions and neutral species) are known by means of which it is possible to perform a bactericidal action and facilitate the precipitation of the suspended dust (through the creation of chemical bonds with ionized particles).

The level of suspended dust suppression and of the inactivations of the bacteria present in the air that is obtained with the devices of the known type is rather limited.

The more recent requirements, due to ever-increasing atmospheric pollution, as well as the health emergency constituted by the COVID-19 pandemic, would suggest the maximum dissemination of devices designed to purify air with a higher performance (which currently do not exist on the market).

The aim of the present invention is to solve the problems described above, proposing an air purifying apparatus, capable of ensuring a high suppression of the dust suspended in the treated air.

Within this aim, an object of the invention is to propose an air purifying apparatus capable of inactivating a significant fraction of the bacteria present in the treated air.

Another object of the invention is to propose an air purifying apparatus capable of inactivating a significant fraction of the viruses present in the treated air.

Another object of the invention is to propose an air purifying apparatus capable of inactivating a significant fraction of the spores present in the treated air.

Another object of the invention is to propose an air purifying apparatus capable of inactivating a significant fraction of the fungi present in the treated air.

Another object of the invention is to propose an air purifying apparatus capable of inactivating a significant fraction of the molds present in the treated air.

Another object of the invention is to propose an air purifying apparatus capable of inactivating a significant fraction of the odors present in the treated air.

Another object of the invention is to propose an air purifying apparatus that has a very simple structure, which therefore can be adapted easily to installation in existing air treatment systems.

Another object of the invention is to propose an air purifying apparatus that ensures operation, albeit with reduced yield, even in the case of failure of one of its components.

Another object of the invention is to propose an air purifying apparatus of moderate dimensions and with a compact shape.

A further object of the present invention is to provide an air purifying apparatus that has low costs, is relatively easy to provide in practice and is of secure application.

This aim and these and other objects that will become more apparent hereinafter are achieved by an air purifying apparatus, of the type comprising a conveyance channel affected by at least one filter, characterized in that it comprises
- at least one first intake filter arranged at the opening for access to said conveyance channel;
- at least one duct provided with an internal coating constituted by a mixture which comprises at least one photocatalytic material;
- at least one LED diode, designed to emit a beam of light that is at least partially comprised in ultraviolet radiation within said duct;
- at least one cold plasma purification device, which has an outlet which leads, even indirectly, to the terminal outflow opening of said channel.

Further characteristics and advantages of the invention will become more apparent from the description of a preferred but not exclusive embodiment of the air purifying apparatus, illustrated by way of non-limiting example in the accompanying drawings, wherein:

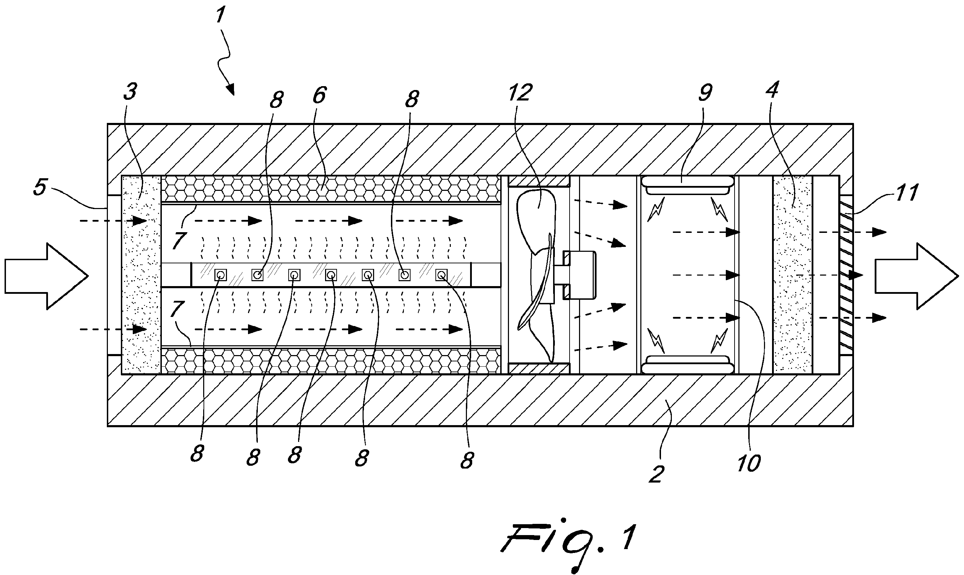

Figure 1 is a schematic view of a possible embodiment of an air purifying apparatus according to the invention.

With reference to the figure, an air purifying apparatus according to the present invention is generally designated by the reference numeral 1.

The apparatus 1 according to the invention comprises a conveyance channel 2 intercepted by at least one filter 3, 4.

In particular, the apparatus 1 comprises at least one first intake filter 3 arranged at the opening 5 for access to the conveyance channel 2.

The apparatus 1 comprises, moreover, at least one duct 6 provided with an internal coating 7 constituted by a mixture which comprises at least one photocatalytic material.

The duct 6 can conveniently intercept the channel 2 by conveying all the circulating air through itself; it is specified that the duct 6 might also advantageously be a portion of the channel 2 on which the coating 7 comprising the photocatalytic material is deposited.

The apparatus 1 according to the invention comprises furthermore advantageously at least one LED diode 8, designed to emit a beam of light that is at least partially comprised in ultraviolet radiation and arranged within the duct 6. The choice of light sources suitable to emit ultraviolet radiation is due to the fact that said radiation has germicidal properties.

The apparatus 1 conveniently comprises, moreover, at least one cold plasma purification device 9, which has an outlet 10 which leads, even indirectly, to the terminal outflow opening 11 of the channel 2.

The device 9 is composed of a power board (which for safety reasons can be preferably chosen from those of the type with low-voltage power supply) capable of supplying an actual plasma generator made of metal alloy and insulating material.

This generator is surrounded by a mesh ring also made of metal alloy. The power board delivers high-voltage current (indicatively between 1,450 V and 1,650 V, although different reference values are not excluded) at a controlled frequency.

The current, by flowing between the generator and the ring, creates a power arc, in the state of plasma, which breaks down some molecules of the air, introducing large quantities of negative ions (indicatively between approximately 4,000 and 9,000 per cm³). The generated ion cloud is then pushed into the environment, creating the beneficial effects described by the available scientific literature.

The physical effect is identical to that of a standard ionizer, but the difference of a plasma generator is of the technological type: much smaller dimensions in relation to the ions generated; greater number of hours of operation, up to 5 times more; total absence of the hum that is typical of grid or lamp ionizing systems; low electric power consumption (it allocates power levels of the order of units or at most a few tens of watts); very low ozone emissions (below 0,05 ppm).

In practice, the air flow passes through the channel 2, encountering at the intake a first filter 3 (through which it is possible to intercept part of the suspended particles and block them); then the air passes through the duct 6, in which the radiation emitted by the LED diodes 8 performs a first germicidal action (inactivating or, in any case, decreasing the vitality of the bacteria that are present). In this duct the light beam emitted by the LED diodes 8 activates the photocatalytic material.

The photocatalytic materials that are usually used are semiconductor materials, due to their particular band-like structure: in fact, in a semiconductor the valence and conduction bands are separated by a band gap in which electrons cannot be present.

When the semiconductor crystal is irradiated by a light source, by means of a beam of light having an energy that is not lower than a threshold value, an electron of the valence band can absorb the energy of the photon and pass to the conduction band, leaving a hole in the valence band.

The electrons and holes thus generated then migrate to the surface of the crystal, where they react with the adsorbed species: electron acceptors or donors. The products that form as a result of this reaction are typically free radicals, highly reactive, which generally then attack other components of the system, such as, in particular, pollutants.

The most frequently used photocatalysts are metallic compounds such as zinc oxide, cerium oxide, zinc sulfide, cadmium sulfide, tin dioxide, zirconium oxide, etcetera.

In industrial applications, the most frequently used material (due to the excellent performance in terms of suppression of pollutants present in the air) is titanium dioxide.

It should also be noted that many photocatalytic materials have a strong bactericidal action.

The pollutants present in the air, therefore, in crossing the duct 6 undergo a synergistic action because they are subjected to the radiation emitted by the LED diodes 8 and to the effects of photocatalysis.

The air then enters the cold plasma purification device 9, where it comes into contact with the generated plasma flow which, through a chemical-physical reaction with the suspended pollutant molecules, suppresses various types of pollutants, including several volatile organic compounds.

It should be highlighted that the air that enters the device 9 originates from the duct 6 and therefore its pollutant content has already been greatly reduced; moreover, the bacteria that are present (if still active) are in any case "weakened" by the previous treatment (exposure to ultraviolet radiation and photocatalysis processes), being therefore easier to inactivate by said device 9.

This synergistic effect ensures a purification performance that is much higher than that expected for purification apparatuses of the known type.

It is possible to provide for the installation of a filter 4 at the outlet of the channel 2 (downstream of the device 9) in order to collect and block the particles and inactivated bacteria during the flow of the air in said channel 2.

It is specified that the mixture used in the coating 7, which comprises at least one photocatalytic material, may advantageously comprise a compound constituted by tin oxide and silicon dioxide in a percentage comprised between 0.5% and 5% by weight, tungsten trioxide in a percentage of no more than 3% by weight, titanium dioxide in a percentage of no more than 3% by weight, organic carbon in a percentage comprised between 0.01% and 0.05% by weight, at least one alcohol in a percentage comprised between 30% and 80% by weight, and water in a percentage comprised between 10% and 60% by weight.

This mixture is particularly active in terms of photocatalysis, since nanometer powders of tungsten trioxide (WO₃) and titanium dioxide (TiO₂), conveniently mixed with the dosages described above, generate a significant synergistic effect, which allows a much higher suppression of pollutants than is obtainable with mixtures of the known type.

The coating 7 that can be obtained with the cited mixture, by virtue of the simultaneous presence (with the dosages indicated previously), ensures the removal of pollutants and microorganisms, combining washability, photocatalytic degradation of pollutants and a strong antimicrobial activity.

The coating 7, by virtue of a particular aggregation ability of titanium dioxide and tungsten trioxide nanopowders, provides an optimization of defects and of crystallinity.

The combination of titanium dioxide and tungsten trioxide inhibits the transformation of titanium dioxide into anatase/rutile (i.e. keeping it in its most active form for photocatalysis); furthermore, the synergistic effect of the two components also induces surface defects that improve the contact angle with water, with consequent inactivation of Escherichia coli bacteria when subjected to light radiation in the visible spectrum.

It is not excluded to adopt nanopowders of titanium dioxide and tungsten trioxide subjected to preventive doping: a highly pure semiconductor at equilibrium has an equal concentration of electrons in the conduction band and holes in the valence band; by adding impurities to the catalyst, however, there is an increase in the concentration of one of the two charge carrier species and therefore an increase in conductivity; in this case one speaks of semiconductor doping.

For example, to make titanium dioxide sensitive to visible light, it is necessary to modify it by means of various methods such as doping with other species or by sensitization with dyes. Similar doping processes can be performed to increase specific chemical-physical properties of titanium dioxide and tungsten trioxide nanopowders.

In order to illustrate possible embodiments of proven effectiveness, it is specified that the at least one alcohol may conveniently be of a type preferably chosen from methyl alcohol, ethyl alcohol, isopropyl alcohol and/or a combination thereof.

It is not excluded to use a different type of alcohol and/or a corresponding combination, different from those cited above.

Moreover, with particular reference to a constructive solution of unquestionable interest in practice and in application, the mixture comprising at least one photocatalytic material may furthermore conveniently comprise silver in a percentage between 0.02% and 0.1% by weight and platinum in a percentage between 0.02% and 0.1% by weight.

Silver and platinum are metals to which undisputed antibacterial properties have been acknowledged for decades: their presence in the coating layer 7 accentuates the suppression of bacteria due to photocatalysis, causing a strong reduction of all the pollutants present in the environment in which the apparatus 1 according to the invention is operating.

According to the invention, it is important to highlight that the coating layer 7 is advantageously deposited directly on the surfaces of the channel 6.

In practice, the described mixture can be deposited directly on the surfaces to be coated without the need to perform pre-treatments with a primer, i.e., in a manner that is totally different from all the solutions of the background art used and tested so far.

This result is achieved by virtue of the presence of organic carbon in the mixture: this definition includes well-defined chemical compounds (such as sugars, fatty acids, hydrocarbons and the like), but also bacteria, microorganisms and the like.

The nature of organic carbon and its tendency to form solid bonds with many different materials make it unnecessary to use a primer (intended to provide a base layer on which mixtures of the known type are normally distributed).

The mixture described above, therefore, by virtue of the presence of organic carbon is capable of adhering to a large number of materials, including wood, plastic, glass, metals, ceramics, cement, interiors and exteriors, construction surfaces.

The adhesion improvement effects ensured by the presence of organic carbon are accentuated by the synergistic effect of this last component with tin oxide and silicon dioxide.

It is specified that the at least one LED diode 8 is of a type designed to emit a beam of type C ultraviolet radiation, having a bactericidal action. The UV-C radiation furthermore activates very effectively the at least one photocatalytic material present in the coating 4, since it has a high energy content and belongs to a frequency band with respect to which photocatalytic materials are particularly reactive.

Ultraviolet germicidal irradiation (UVGI in acronym) is an electromagnetic radiation with wavelengths shorter than those of light. UV can be divided into several categories, the short category (UV-C) is considered "germicidal UV".

At certain wavelengths, UV is harmful to bacteria, viruses and other microorganisms. At a wavelength of 2537 Angstrom (254 nm), UV destroys the molecular bonds of the DNA of microorganisms, producing thymine dimers in their DNA and destroying them, making them harmless or preventing their growth and reproduction.

It is a process similar to the effect of longer-wavelength UV (UVB) on humans, for example sunburns or the blinding effect of light. Microorganisms have little protection from UV and cannot survive a prolonged exposure.

UVGI is a very effective method of destroying microorganisms. The effectiveness depends on many factors: the amount of exposure time, the power variations of the UV source which affects the electromagnetic wavelength, the presence of particles that can protect microorganisms from UV, and the ability of the microorganisms to resist the radiation during exposure. In many systems, the effectiveness is increased by repeated circulation of the water or air, to increase the probability that the ultraviolet radiation will strike the microorganisms and to irradiate them several times.

The effectiveness of this sterilization method also depends on the configuration of the environment: an environment in which there are obstacles to the light of the UV lamp is not effective. In these cases, the effectiveness depends on the point where the UV lamp is positioned. An increase in effectiveness can be achieved by using reflection. The adoption of surfaces inside the duct 6 with a high reflection rate are very useful to reflect ultraviolet radiation, amplifying its effects.

If the apparatus 1 is not integrated within an air conditioning or ventilation system or device, it is specified that it must advantageously comprise at least one forced ventilation assembly 12 to establish an air flow between the access opening 5 to the conveyance channel 2 and the terminal outflow opening 11 of said channel 2.

With particular reference to a constructive solution of unquestionable interest in application and suitable to achieve a very high purification performance, it is specified that at least one component chosen from the structural frame of the at least one filter 3, 4, the filtering surfaces of the at least one filter 3, 4, the at least one forced ventilation assembly 12, and the supporting framework of the at least one cold plasma purification device 9 may advantageously be provided with a surface coating constituted by a mixture which comprises at least one photocatalytic material.

The mixture may be of the type of the one described previously, although the possibility to use a different mixture to provide the coating of components other than the internal surfaces of the duct 6 (on which the coating 7 made with the mixture described previously is deposited) is not excluded.

It is furthermore highlighted that the at least one filter 3, 4 may conveniently be of a type preferably chosen from filters made of textile fibers with a filtration capacity up to 20 µm, filters made of natural fibers with a filtration capacity up to 20 µm, HEPA (High Efficiency Particulate Air) filters, with a filtration capacity up to 0.3 µm, activated carbon filters, filters at least partially coated with mixtures comprising photocatalytic materials, ceramic filters, HVAC (Heating, Ventilation and Air Conditioning) filters, fiberglass filters, polyester filters, paper and paper-derivative filters, metal filters, polymer foam filters, polymer sponge filters, filters constituted by combinations at least of pairs of those previously mentioned.

In particular, it is specified, by way of non-limiting example, that the intake filter 3 might be constituted by a series of filters arranged in cascade.

For example, it might comprise a cotton pre-filter to remove impurities larger than 20 µm, an HEPA filter in order to remove very small impurities, an activated carbon filter in order to preliminarily absorb odors, and a filter treated with a photocatalyst.

Different configurations for the intake filter 3 and the outlet filter are not excluded, and the insertion of further filters, interposed between the other components of the apparatus 1, also is not excluded.

The present invention extends its protection also to an air treatment system comprising at least one climate control unit that intercepts a duct for distributing air in a predefined number of environments (of the type of a ventilation system, air conditioning system, heating system and the like).

The system according to the invention usefully comprises, along the duct, an air purification apparatus 1 as described earlier.

In particular, the apparatus 1 comprises favorably a conveyance channel 2 intercepted by at least one filter 3, 4 and comprising at least one first intake filter 3 arranged at the opening 5 for access to the conveyance channel 2, at least one duct 6 provided with an internal coating 7 constituted by a mixture which comprises at least one photocatalytic material, at least one LED diode 8 designed to emit a beam of light that is at least partially comprised in ultraviolet radiation within the duct 6, and at least one cold plasma purification device 9, which has an outlet 10 which leads, even indirectly, to the terminal outflow opening 11 of the channel 2.

In this way the air, before or after undergoing the treatment provided by the present climate control unit, undergoes a thorough purification process, so that it can be conveyed along the duct and spread to the served environments with a minimum load of pollutants.

The channel 2, as well as the duct 6, may have any shape depending on the specific requirements, and the materials used to provide the various components cited may be of any type. The choice of materials with high reflectance makes it possible to maximize the performance of the germicidal action of UV-C radiation emitted by the LED diodes 8 and the suppression of pollutants provided by photocatalysis.

The coating 7 (and also any coating of the other components) may be performed by means of a spray process, known as "spray coating", plasma distribution, known as "plasma coating", and the distribution known as "deep coating".

The number, distribution and power of the LED diodes 8 installed shall depend on the dimensions of the channel 6 and on the germicidal action they are required to have.

The ventilation assemblies 12 may also vary in number according to the specific requirements (when necessary) and have a power suitable to generate the expected air flow rate.

The air that is introduced in the environment downstream of the apparatus 1 is purified from pollutants, dust, odors, vapors, and microorganisms (such as bacteria, germs, and the like), to the full benefit of the well-being of the people who are in the environment in which the apparatus 1 operates.

Advantageously, the present invention solves the problems described above, proposing an air purifying apparatus 1 capable of ensuring a strong suppression of dust in suspension in the treated air.

Conveniently, the apparatus 1 according to the invention is capable of inactivating a significant fraction of the bacteria present in the treated air.

Positively, the apparatus 1 according to the invention has a very simple structure and is therefore easily adaptable to installation in existing air treatment systems.

Positively, the apparatus 1 according to the invention ensures operation, albeit with reduced yield, even in the event of failure of one of its components (in fact, it is still possible to benefit from the pollutant suppression effects induced by the components that are still regularly active).

Efficiently, the apparatus 1 according to the invention may also have small dimensions and a compact shape. By virtue of this embodiment, it will also be possible to provide apparatuses 1 intended for domestic application. In this case, the shape and dimensions will be chosen according to specific design requirements.

Effectively, the apparatus 1 according to the invention is found to be capable of inactivating a significant fraction of the viruses present in the treated air.

Usefully, the apparatus 1 according to the invention is found to be capable of inactivating a significant fraction of the spores present in the treated air.

Favorably, the apparatus 1 according to the invention is found to be capable of inactivating a significant fraction of the fungi present in the treated air.

Advantageously, the apparatus 1 according to the invention is found to be capable of inactivating a significant fraction of the molds present in the treated air.

Conveniently, the apparatus 1 according to the invention is found to be capable of inactivating a significant fraction of the odors present in the treated air.

Validly, the air purifying apparatus 1 is relatively simple to provide in practice and has low costs: these characteristics make the apparatus 1 according to the invention an innovation of secure application.

The invention thus conceived is susceptible of numerous modifications and variations, all of which are within the scope of the appended claims ; all the details may furthermore be replaced with other technically equivalent elements.

In the exemplary embodiments shown, individual characteristics, given in relation to specific examples, may actually be interchanged with other different characteristics that exist in other exemplary embodiments.

In practice, the materials used, as well as the dimensions, may be any according to the requirements and the state of the art.

The disclosures in Italian Patent Application No. 102020000019462 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An air purifying apparatus, of the type comprising a conveyance channel (2) intercepted by at least one filter (3, 4), **characterized in that** it comprises
- at least one first intake filter (3) arranged at the opening (5) for access to said conveyance channel (2);
- at least one duct (6) provided with an internal coating (7) constituted by a mixture which comprises at least one photocatalytic material;
- at least one LED diode (8), designed to emit a beam of light that is at least partially comprised in ultraviolet radiation within said duct (6);
- at least one cold plasma purification device (9), which has an outlet (10) which leads, even indirectly, to the terminal outflow opening (11) of said channel (2).

2. The apparatus according to claim 1, **characterized in that** said mixture comprising at least one photocatalytic material comprises
- a compound constituted by tin oxide and silicon dioxide in a percentage comprised between 0.5% and 5% by weight,
- tungsten trioxide in a percentage of no more than 3% by weight,
- titanium dioxide in a percentage of no more than 3% by weight,
- organic carbon in a percentage comprised between 0.01% and 0.05% by weight,
- at least one alcohol in a percentage comprised between 30% and 80% by weight,
- water in a percentage comprised between 10% and 60% by weight.

3. The apparatus according to claim 2, **characterized in that** said mixture comprising at least one photocatalytic material comprises
- silver in a percentage comprised between 0.02% and 0.1% by weight,
- platinum in a percentage comprised between 0.02% and 0.1% by weight.

4. The apparatus according to claim 1, **characterized in that** said coating layer (7) is deposited directly on the internal surfaces of said duct (6), without the interposition of a primer.

5. The apparatus according to claim 1, **characterized in that** at least one filter (4) is interposed between the outlet of said at least one cold plasma purification device (9) and the terminal outflow opening (11) of said channel (2).

6. The apparatus according to claim 1, **characterized in that** said at least one LED diode (8) is designed to emit a beam of type C ultraviolet radiation, which has a bactericidal action, said UV-C radiation activating the at least one photocatalytic material that is present in said coating (7).

7. The apparatus according to one or more of the preceding claims, **characterized in that** it comprises at least one forced ventilation assembly (12) for establishing a flow of air between the opening (5) for access to said conveyance channel (2) and the terminal outflow opening (11) of said channel (2).

8. The apparatus according to one or more of the preceding claims, **characterized in that** at least one component chosen between the structural frame of said at least one filter (3, 4), the filtering surfaces of said at least one filter (3, 4), said at least one forced ventilation assembly (12), the supporting framework of said at least one cold plasma purification device (9) is provided with a surface coating constituted by a mixture which comprises at least one photocatalytic material.

9. The apparatus according to claim 1, **characterized in that** said at least one filter (3, 4) is of the type preferably chosen from:
- filters made of textile fibers with a filtering capacity up to 20 µm;
- filters made of natural fibers with a filtration capacity up to 20 µm;
- HEPA (High Efficiency Particulate Air) filters, with a filtration capacity up to 0.3 µm;
- activated carbon filters;
- filters at least partially coated with mixtures comprising photocatalytic materials;
- ceramic filters;
- HVAC (Heating, Ventilation and Air Conditioning) filters;
- glass fiber filters;
- polyester filters;
- filters made of paper and paper derivatives;
- metal filters;
- polymeric foam filters;
- polymeric sponge filters;
- filters constituted by combinations at least of pairs of those mentioned above.

10. An air treatment system, comprising at least one climate control unit which intercepts a duct for distributing air into a predefined number of environments, **characterized in that** it comprises, along said duct, an air purifying apparatus (1) which comprises a conveyance channel (2) intercepted by at least one filter (3, 4) and comprising at least one first intake filter (3) arranged at the opening (5) for access to said conveyance channel (2), at least one duct (6) provided with an internal coating (7) constituted by a mixture which comprises at least one photocatalytic material, at least one LED diode (8) designed to emit a beam of light that is at least partially comprised in ultraviolet radiation within said duct (6), and at least one cold plasma purification device (9), which has an outlet (10) which leads, even indirectly, to the terminal outflow opening (11) of said channel (2).
